# FASCICULE DE BREVET EUROPEEN

(11) **EP 1 436 027 B1**
(45) Date de publication et mention de la délivrance du brevet: **22.08.2007**
(21) Numéro de dépôt: 02801368.8
(22) Date de dépôt: 15.10.2002
(51) Int. Cl.: A61M 5/32, A61M 5/50

(54) **DISPOSITIF DE SECURITE POUR UNE SERINGUE**
SICHERHEITSVORRICHTUNG FÜR EINE SPRITZE
SAFETY DEVICE FOR A SYRINGE

(30) Priorité: 15.10.2001 FR 0113255
(43) Date de publication de la demande: 14.07.2004
(73) Titulaire: Rexam Pharma La Verpillière, 38290 La Verpilliere Cedex (FR)
(72) Inventeur: RIMLINGER, Thierry, F-38080 L'Isle d'Abeau (FR); POUGET, Michel, F-38300 Domarin (FR); DODIER, Philippe, F-69110 Sainte Foy les Lyon (FR)
(74) Mandataire: Remy, Vincent Noel Paul
(86) Numéro de dépôt international: PCT/FR2002/003531
(87) Numéro de publication internationale: WO 2003/033060

(56) Documents cités:
- EP-A- 0 317 518
- WO-A-01/85239
- WO-A-89/04681
- DE-A- 3 843 839
- FR-A- 2 788 986
- FR-A- 2 815 545

## Description

L'invention a pour objet un dispositif de sécurité pour une seringue, notamment pour une seringue pré-remplie, comportant un fourreau de protection permettant de protéger l'aiguille de la seringue après son utilisation.

On connaît par le brevet européen EP 0 317 518 un dispositif de protection d'une aiguille de seringue comprenant un étui de protection et une bague élastique comportant deux prolongements coaxiaux diamétralement opposés, l'un des prolongements faisant partie intégrante d'une extrémité de l'étui de protection et l'autre prolongement faisant partie intégrante d'un écrou relié à une extrémité du corps de la seringue. La structure de ce dispositif de protection connu n'est adaptée qu'à un seul type de seringue et la mise en place de ce dispositif sur la seringue est relativement complexe.

Il existe un besoin de disposer d'un dispositif de sécurité fiable, notamment adapté à différents types de seringues, permettant de réduire le plus possible les risques de contamination accidentelle.

Il existe également un besoin de disposer d'un tel dispositif de sécurité présentant une structure relativement simple et pouvant être mis en place de manière aisée sur la seringue.

L'invention a pour objet un dispositif de sécurité pour une seringue, cette dernière comportant un corps, un porte-aiguille à une extrémité de ce corps, un piston mobile dans le corps et une tige de piston dépassant du corps à l'opposé du porte-aiguille et apte à pousser le piston dans le corps en direction du porte-aiguille, ledit dispositif comportant un fourreau de protection dans lequel le corps de la seringue peut coulisser entre une position d'injection dans laquelle l'aiguille de la seringue s'étend à l'extérieur du fourreau de protection et une position de sécurité dans laquelle l'aiguille est en retrait à l'intérieur du fourreau de protection, ce dispositif étant caractérisé par le fait qu'il comporte un élément élastique apte à agir sur la seringue pour l'amener dans sa position de sécurité après injection du produit de la seringue, cet élément élastique comportant deux extrémités axiales et au moins deux bras élastiquement déformables s'étendant entre ces deux extrémités axiales, l'une des extrémités étant agencée de manière à pouvoir coulisser librement par rapport à la tige de piston avant l'injection, et/ou l'une des extrémités étant agencée de manière à pouvoir être fixée au fourreau de protection dans une position réglable en hauteur.

L'invention est particulièrement bien adaptée aux seringues pré-remplies et est notamment destinée à un usage unique.

Grâce à l'invention, les bras élastiquement déformables ne sont pas précontraints avant l'utilisation de la seringue, soit parce que les bras élastiques sont dans leur état de repos, après la mise en place de l'élément élastique sur la tige de piston, l'une des extrémités de l'élément élastique étant libre, soit parce que la distance entre les deux extrémités axiales de l'élément élastique a été adaptée aux dimensions de la seringue grâce au réglage précité.

Ainsi, les bras élastiquement déformables conservent leur capacité de retour élastique, même après un stockage prolongé du dispositif de sécurité et après injection du contenu de la seringue. Les bras sont ainsi en mesure d'exercer une action élastique suffisante pour amener le corps de la seringue dans la position de sécurité dans laquelle l'aiguille est en retrait à l'intérieur du fourreau de protection.

Par ailleurs, lorsque l'une des extrémités axiales de l'élément élastique est agencée de manière à pouvoir coulisser librement par rapport à la tige de piston, la longueur commune des bras élastiquement déformables peut être choisie de manière à ce que l'élément élastique soit adapté à différents types de seringues, cette longueur étant inférieure à celle des tiges de piston de différents types de seringues. Lorsque l'une des extrémités est agencée de manière à pouvoir être fixée au fourreau de protection dans une position réglable en hauteur, on peut adapter ce réglage aux dimensions de la seringue à protéger. Ainsi le dispositif de sécurité selon l'invention est adapté à différents types de seringues, notamment des seringues de 0,5 ml et de 1 ml, ce qui permet de n'avoir à disposer que d'un seul type de dispositif de sécurité pour toute une gamme de seringues.

Outre le fait qu'il est adapté à plusieurs types de seringues, le dispositif de sécurité peut également permettre de protéger des seringues d'un même type mais dont le dosage est variable, c'est-à-dire que la course initiale du piston peut être réglée selon la dose de produit à injecter.

Enfin, les bras élastiques remplacent avantageusement un ressort hélicoïdal par exemple, ce qui permet d'avoir un nombre réduit de pièces constitutives et de réduire le coût de revient du dispositif de sécurité.

Dans une mise en oeuvre préférée de l'invention, les bras élastiquement déformables sont définis chacun par une bande de matière, réalisée par exemple en PP, SBS, EBS, PA, POM, PE, cette liste n'étant pas limitative. Ces bras sont de préférence au nombre de deux et disposés en regard l'un de l'autre.

Le fourreau peut être réalisé dans une matière transparente ou semi-transparente.

Les bras présentent avantageusement chacun une forme générale en V, de concavité dirigée vers l'intérieur. Cette forme générale en V permet d'obtenir de bonnes propriétés de retour élastique des bras.

Dans une mise en oeuvre préférée de l'invention, les branches du V de chacun des bras présentent des longueurs différentes, améliorant encore les propriétés de retour élastique de ces bras.

Ces bras peuvent présenter localement une largeur réduite, permettant de les écarter de manière à ce que l'élément élastique ne gêne pas la mise en place de la seringue dans le fourreau de protection.

Dans une mise en oeuvre particulière de l'invention, l'élément élastique est réalisé d'un seul tenant avec le fourreau de protection, permettant ainsi de réduire le nombre de pièces constitutives du dispositif de sécurité.

Selon un aspect de l'invention, l'une des extrémités de l'élément élastique se raccorde au fourreau de protection et l'autre extrémité est mise en place sur la tige de piston en étant libre de coulisser par rapport à celle-ci, avant l'injection.

L'élément élastique peut alors comporter à une extrémité axiale une bague annulaire fendue apte à s'engager sur la tige de piston et pouvant coulisser par rapport à celle-ci.

La mise en place de la seringue dans le dispositif de sécurité est ainsi simple et rapide, la bague fendue étant notamment engagée sur la tige de piston par un geste simple de pression.

En variante, l'élément élastique est constitué par un élément rapporté apte à être fixé sur le fourreau de protection.

L'élément élastique peut alors comporter des lanières de fixation percées chacune d'une pluralité d'orifices suivant une direction longitudinale de la lanière et le fourreau de protection comporter des reliefs associés chacun à une lanière, chaque relief pouvant être fixé dans un des orifices de la lanière associée. Ainsi, on peut régler en hauteur la position de fixation des lanières sur le fourreau de protection.

De plus, l'assemblage du dispositif de sécurité et de la seringue est relativement aisé, nécessitant simplement l'introduction de la seringue dans le fourreau puis la fixation des lanières à celui-ci.

L'extrémité de l'élément élastique opposée aux lanières peut être solidaire de la tige de piston de la seringue.

Le dispositif comporte avantageusement un élément de maintien apte à passer par déformation d'une position de maintien du corps de la seringue dans le fourreau à une position dégagée permettant au corps de la seringue de coulisser librement par rapport au fourreau.

Cet élément de maintien permet notamment d'immobiliser en translation le corps de seringue par rapport au fourreau de protection lors de l'introduction de l'aiguille sous la peau du patient.

Cet élément de maintien peut comporter des pattes élastiquement déformables réalisées notamment sur le fourreau de protection et aptes à être écartées pour prendre la position dégagée.

Dans une mise en oeuvre particulière de l'invention, l'élément élastique comporte des ponts de matière reliant les bras élastiquement déformables à ces pattes de manière à ce que, lorsque l'élément élastique subit une compression, ces ponts de matière tendent à écarter les pattes.

En variante, l'élément élastique comporte une nervure annulaire agencée pour venir en appui sur lesdites pattes et les écarter lorsque l'élément élastique subit une compression.

Ainsi, l'élément de maintien est dégagé suite à la course de la tige de piston lors de l'injection, ce qui évite à l'utilisateur d'avoir à effectuer un geste supplémentaire autre que celui d'une injection classique pour dégager cet élément de maintien.

Le dispositif de sécurité peut encore comporter un élément de verrouillage apte à être fixé sur le corps de la seringue et agencé pour retenir celui-ci dans sa position de sécurité, après l'injection, cet élément de verrouillage pouvant comporter des dents aptes à venir en butée contre un bord supérieur du fourreau de protection lorsque le corps de la seringue est dans cette position de sécurité.

Le dispositif de sécurité peut également comporter des reliefs permettant d'immobiliser en rotation la seringue par rapport au fourreau de protection.

L'invention sera mieux comprise à la lecture de la description détaillée qui va suivre, d'exemples de mise en oeuvre non limitatifs, en référence au dessin annexé, sur lequel :
- la figure 1 représente schématiquement et partiellement, en coupe axiale, une seringue équipée d'un dispositif de sécurité conforme à un premier exemple de mise en oeuvre de l'invention, avant l'utilisation de la seringue,
- la figure 2 représente schématiquement et partiellement, en perspective, le fourreau de protection et l'élément élastique du dispositif de la figure 1,
- la figure 3 représente schématiquement, en perspective, un manchon du dispositif de sécurité de la figure 1 destiné à recevoir le corps de la seringue,
- la figure 4 est une vue de côté d'une variante du dispositif de sécurité de la figure 1,
- la figure 5 est une vue analogue à celle de la figure 1, après injection,
- la figure 6 est une coupe schématique suivant VI-VI du dispositif de la figure 5,
- la figure 7 représente schématiquement et partiellement, en coupe axiale, un dispositif de sécurité conforme à un deuxième exemple de mise en oeuvre de l'invention,
- la figure 8 est une vue schématique de côté d'un dispositif de sécurité conforme à un troisième exemple de mise en oeuvre de l'invention,
- la figure 9 est une coupe axiale schématique suivant IX-IX du dispositif de la figure 8, et
- la figure 10 représente schématiquement et partiellement, en coupe axiale, un dispositif de sécurité conforme à un quatrième exemple de mise en oeuvre de l'invention.

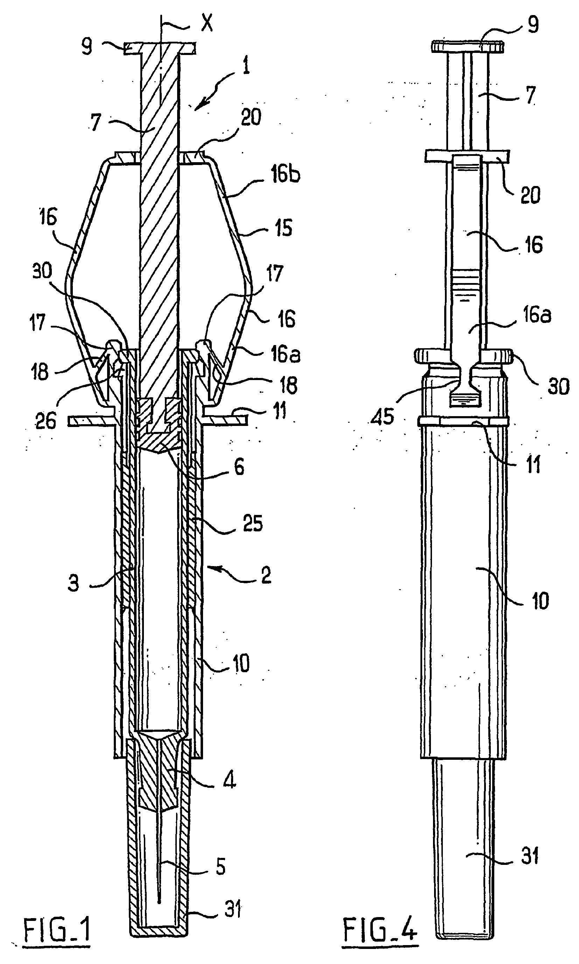

On a représenté sur la figure 1 une seringue 1 équipée d'un dispositif de sécurité 2 destiné à un usage unique conforme à l'invention.

La seringue 1 comporte, de manière connue en soi, un corps 3, un porte-aiguille 4 à une extrémité de ce corps 3 et muni d'une aiguille 5, un piston 6 mobile dans le corps 3 et une tige de piston 7 dépassant du corps 3 à l'opposé du porte-aiguille 4 et apte à pousser le piston 7 dans le corps 3 en direction du porte-aiguille 4. Cette tige 7 se termine par un poussoir 9 sur lequel un utilisateur peut exercer une pression pour procéder à l'injection du contenu de la seringue. Le corps 3 présente à son extrémité supérieure une collerette 30.

Le dispositif de sécurité 2 comporte un fourreau de protection 10 cylindrique de révolution d'axe X, comportant à proximité de son extrémité supérieure une collerette 11 définissant un appui pour les doigts d'un utilisateur procédant à une injection.

Le dispositif de sécurité 2 comporte également un élément élastique 15 comprenant deux bras élastiquement déformables 16 définis chacun par une bande de matière présentant une forme générale en V, de concavité dirigée vers l'intérieur, c'est-à-dire vers l'axe X, et en regard l'un par rapport à l'autre.

L'élément élastique 15 est réalisé d'un seul tenant avec le fourreau de protection 10.

Dans l'exemple décrit, la branche inférieure 16a du bras 16 présente une longueur inférieure à la branche supérieure 16b, améliorant ainsi la capacité de retour élastique du bras 16.

Le fourreau de protection 10 comporte à son extrémité supérieure, au-dessus de la collerette 11, deux pattes élastiquement déformables 17 reliées chacune à la branche inférieure 16a d'un bras 16 par l'intermédiaire d'un pont de matière 18.

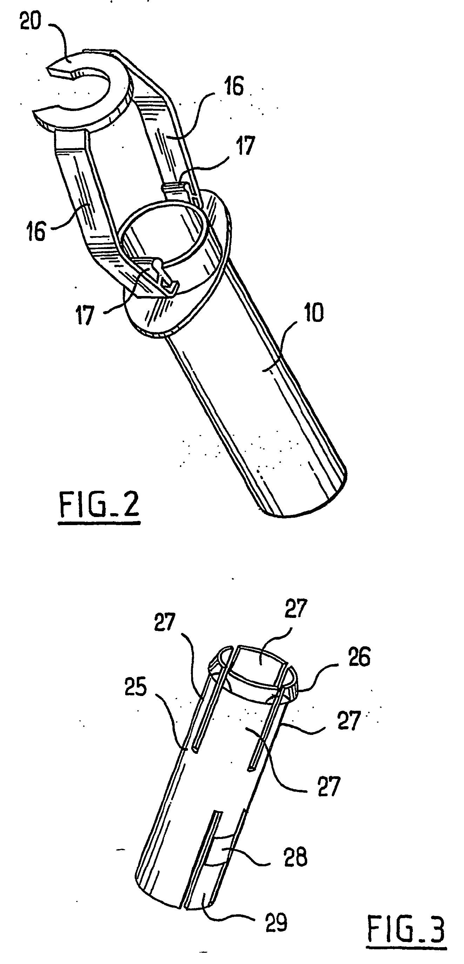

Les bras 16 se raccordent à leur extrémité supérieure à une bague fendue 20, comme on peut le voir sur la figure 2 notamment. Cette bague fendue 20 est destinée à s'engager autour de la tige de piston 7, le diamètre intérieur de cette bague 20 étant supérieur à la section de la tige 7 de sorte qu'elle puisse coulisser librement le long de la tige 7. La hauteur des bras 16 est choisie de manière à ce que la bague 20 reste en dessous du poussoir 9 de la tige 7, permettant ainsi aux bras 16 de ne pas être précontraints avant l'utilisation de la seringue.

On fixe autour du corps de seringue 3 un manchon 25 cylindrique de révolution d'axe X. Ce manchon 25, représenté isolément à la figure 3, comporte une pluralité de pattes élastiquement déformables 27, au nombre de quatre dans l'exemple illustré mais pouvant également être au nombre de six, dont les bords supérieurs définissent un bourrelet annulaire 26. Ce manchon 25 présente sur sa partie inférieure une pluralité de dents 28, au nombre de trois dans l'exemple décrit, réalisées chacune sur une patte élastiquement déformable 29.

Ce manchon 25 reste solidaire du corps de seringue 3 par friction et forme notamment un élément de verrouillage au sens de la présente invention, comme cela va être expliqué plus loin.

Les pattes élastiquement déformables 27 permettent en outre au manchon 25, du fait d'un léger écartement de celles-ci, d'accueillir des corps de seringue de diamètres différents.

Lorsque le manchon 25 est en place dans le fourreau de protection 10, le bourrelet annulaire 26 vient en appui sur le bord supérieur de ce fourreau de protection 10. La collerette 30 de la seringue repose sur ce bourrelet 26 et y est maintenu au moyen des pattes 17. Ainsi, tant que l'élément élastique 15 est dans son état de repos, avant l'injection, comme représenté sur la figure 1, le manchon 25 et le corps de seringue 3 sont immobilisés en translation par rapport au fourreau de protection 10.

Avant l'utilisation de la seringue, l'aiguille 5 est avantageusement protégée par un capuchon 31.

Le fonctionnement du dispositif de sécurité 2 s'effectue de la manière suivante.

D'abord, l'utilisateur fait pénétrer l'aiguille 5 sous la peau du patient, la seringue étant alors immobilisée en translation par rapport au fourreau 10, comme précisé plus haut.

Puis, l'utilisateur enfonce le poussoir 9 de manière à injecter le contenu de la seringue. Lors de cette opération, le poussoir 9 vient en appui sur la bague annulaire 20 et provoque la compression des bras 16. Les branches 16a se plient alors vers l'extérieur de sorte que les pattes 17 soient écartées par l'intermédiaire des ponts de matière 18, permettant ainsi au corps de seringue 3 de ne plus être maintenu contre le fourreau de protection 10.

En fin de course, le piston 6 est retenu par friction dans le corps de seringue 3 de sorte que la tige de piston 7 et le corps 3 soient solidaires. Lorsque l'utilisateur relâche la pression sur le poussoir 9, le retour élastique des bras 16 provoque, par la poussée exercée par la bague 20 sur le poussoir 9, le coulissement vers le haut de l'ensemble constitué de la seringue 1 et du manchon 25 par rapport au fourreau de protection 10. Au terme de ce coulissement, l'aiguille 5 est complètement en retrait à l'intérieur du fourreau de protection 10, dans sa position de sécurité.

On note que ce coulissement correspond à un mouvement de recul de la seringue dans le fourreau de protection 10.

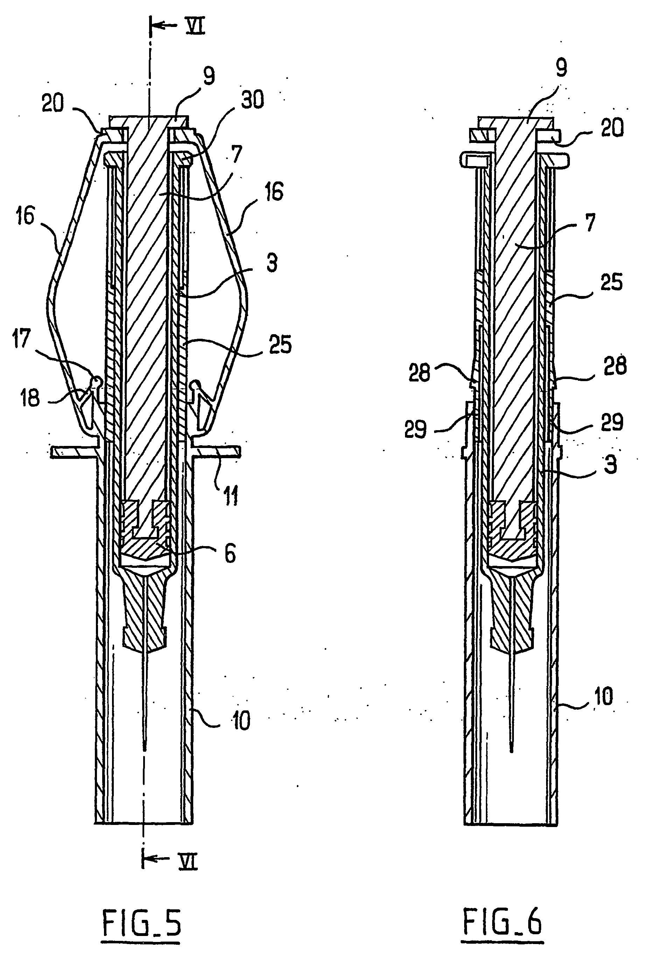

Les dents 28 du manchon 25 se trouvent alors à l'extérieur du fourreau de protection 10 et, par retour élastique des pattes 29 vers l'extérieur, ces dents 28 peuvent venir en butée sur le bord supérieur du fourreau de protection 10 de manière à empêcher l'ensemble constitué du manchon 25 et de la seringue 1 de coulisser en direction du fourreau de protection 10, comme on peut le voir sur la figure 6. On obtient ainsi le verrouillage dans cette position de sécurité de la seringue 1 par rapport au fourreau de protection 10.

Comme on peut le voir sur les figures 5 et 6, à la fin de l'injection, la bague 20 est en appui contre le poussoir 9, au-dessus de la collerette 30.

Les bras 16 comportent localement un rétrécissement de largeur 45 permettant de les écarter de l'axe X de manière à ménager un espace suffisant pour introduire la seringue 1 dans le fourreau de protection 10, comme on peut le voir sur la figure 4.

Les pattes 17 peuvent être écartées par des éléments autres que les ponts de matière 18.

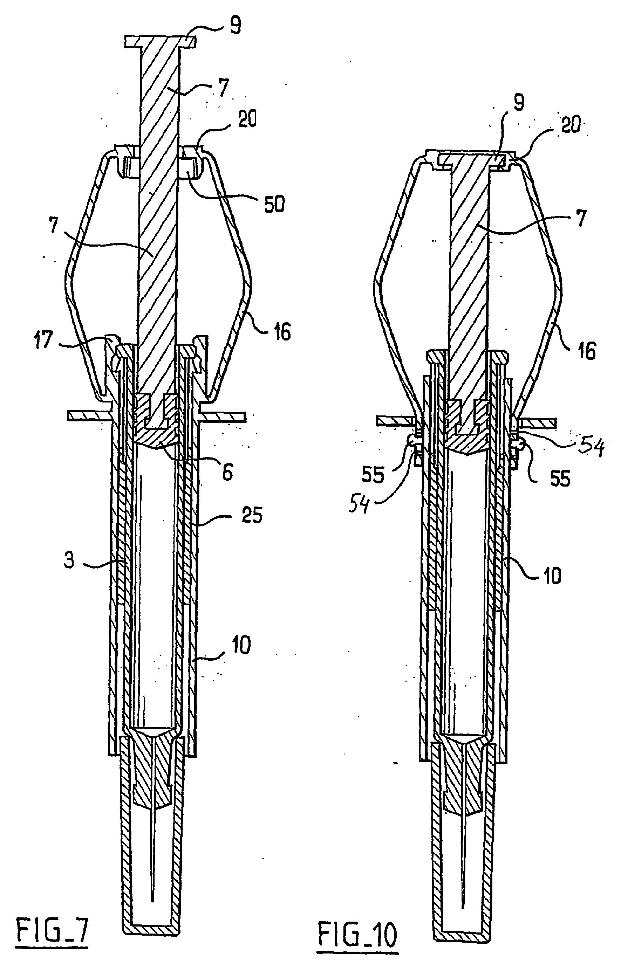

On a représenté à la figure 7 un dispositif de sécurité sensiblement analogue au dispositif 1, mais dépourvu de ponts de matière 18, et comportant une nervure annulaire 50 se raccordant inférieurement à la bague 20. Lorsque le piston 6 est en fin de course, cette nervure annulaire 50 vient en appui sur les pattes 17 et provoque leur écartement de manière à ce que ces dernières ne retiennent plus le corps de la seringue 3 dans le fourreau de protection 10.

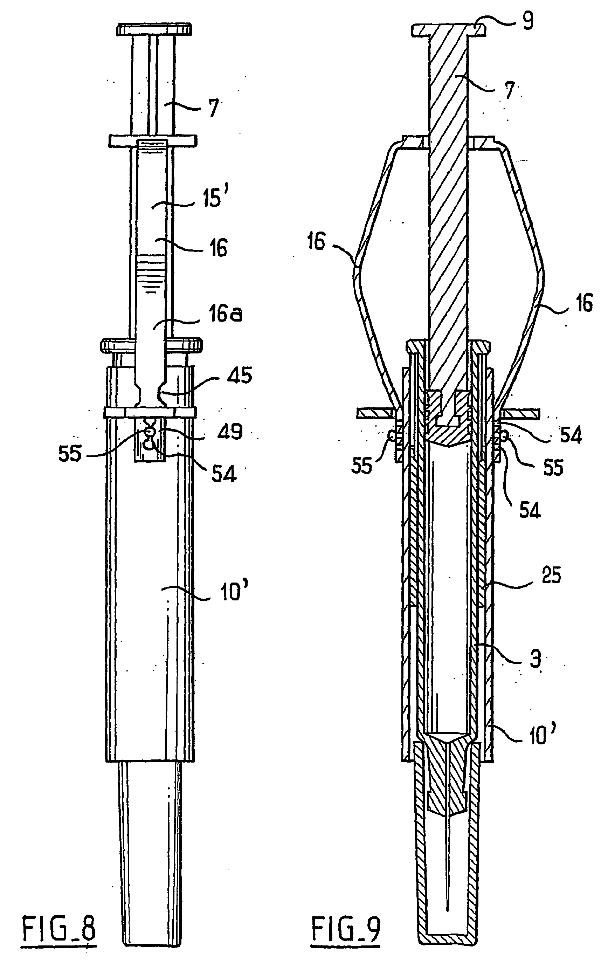

On a représenté sur les figures 8 et 9 un dispositif de sécurité conforme à un autre exemple de mise en oeuvre de l'invention. Comme on peut le voir sur la figure 8, ce dispositif de sécurité comporte un élément élastique 15' se différenciant de l'élément élastique 15 précédemment décrit par le fait qu'il est constitué par un élément rapporté. Les branches inférieures 16a des bras 16 sont prolongées chacune par une lanière 49 percée d'une pluralité de trous disposés suivant une direction longitudinale du bras 16 correspondant.

Le fourreau de protection 10' comporte des reliefs en forme de tétons 55 diamétralement opposés aptes à coopérer chacun avec un orifice 54 choisi parmi les orifices d'un bras 16 en fonction de la hauteur souhaitée de l'élément élastique 15'.

On a représenté sur la figure 10 un dispositif de sécurité sensiblement analogue à celui décrit aux figures 8 et 9 mais se différenciant de celui-ci par le fait que le poussoir 9 est solidarisé à la bague 20, par encliquetage de ce poussoir 9 dans une gorge de la bague 20. Le réglage en hauteur de l'élément élastique 15 est alors effectué en sélectionnant les orifices 54 adaptés dans lesquels s'engagent les reliefs 55.

Bien entendu, l'invention n'est pas limitée aux exemples de mise en oeuvre qui viennent d'être décrits.

On peut notamment prévoir une tige de piston 7 comportant déjà les bras élastiquement déformables 16, lesquelles se raccordent directement au poussoir 9.

Le dispositif de sécurité peut encore comporter des reliefs permettant d'immobiliser en rotation la seringue par rapport au fourreau de protection.

Le fourreau peut également comporter des ajours permettant la préhension de l'ensemble constitué de la seringue et du manchon par un utilisateur.

## Revendications

1. Dispositif de sécurité (2) pour une seringue (1) pré-remplie, cette dernière comportant un corps (3), un porte-aiguille (4) à une extrémité de ce corps, un piston mobile (6) dans le corps et une tige de piston (7) dépassant du corps à l'opposé du porte-aiguille et apte à pousser le piston dans le corps en direction du porte-aiguille, ledit dispositif comportant un fourreau de protection (10) dans lequel le corps de la seringue peut coulisser entre une position d'injection dans laquelle l'aiguille de la seringue s'étend à l'extérieur du fourreau de protection et une position de sécurité dans laquelle l'aiguille est en retrait à l'intérieur du fourreau de protection, **caractérisé par le fait que** le dispositif (2) comporte un élément élastique (15) apte à agir sur la seringue pour l'amener dans sa position de sécurité après injection, cet élément élastique comportant deux extrémités axiales et au moins deux bras élastiquement déformables s'étendant entre ces deux extrémités axiales, l'une des extrémités (20) étant agencée de manière à pouvoir coulisser librement par rapport à la tige de piston (7) avant l'injection, et/ou l'une des extrémités étant agencée de manière à pouvoir être fixée au fourreau de protection (10) dans une position réglable en hauteur.

2. Dispositif selon la revendication 1, **caractérisé par le fait que** les bras élastiquement déformables (16) sont définis chacun par une bande de matière, réalisée par exemple en PP, SBS, EBS, PA, POM, PE, et sont de préférence au nombre de deux et disposés en regard l'un de l'autre.

3. Dispositif selon la revendication 2, **caractérisé par le fait que** lesdits bras (16) présentent chacun une forme générale en V, de concavité dirigée vers l'intérieur.

4. Dispositif selon la revendication 3, **caractérisé par le fait que** les branches du V de chacun des bras (16) présentent des longueurs différentes.

5. Dispositif selon l'une quelconque des revendications 2 à 4, **caractérisé par le fait que** les bras (16) présentent localement une largeur réduite.

6. Dispositif selon l'une quelconque des revendications précédentes, **caractérisé par le fait que** l'élément élastique (15) est réalisé d'un seul tenant avec le fourreau de protection (10).

7. Dispositif selon la revendication 6, **caractérise par le fait que** l'une des extrémités de l'élément élastique se raccorde au fourreau de protection (10) et l'autre extrémité est mise en place sur la tige de piston (7) en étant libre de coulisser par rapport à celle-ci, avant l'injection.

8. Dispositif selon l'une des revendications 6 ou 7, **caractérisé par le fait que** l'élément élastique (15) comporte, à une extrémité axiale, une bague annulaire fendue (20) apte à s'engager sur la tige de piston (7) et pouvant coulisser par rapport à celle-ci.

9. Dispositif selon l'une quelconque des revendications 1 à 5, **caractérisé par le fait que** l'élément élastique (15) est constitué par un élément rapporté apte à être fixé sur le fourreau de protection (10).

10. Dispositif selon la revendication 9, **caractérisé par le fait que** l'élément élastique (15) comporte des lanières de fixation (49) percées chacune d'une pluralité d'orifices suivant une direction longitudinale de la lanière et le fourreau de protection comporte des reliefs (55) associés chacun à une lanière, chaque relief pouvant être fixé dans un des orifices de la lanière associée.

11. Dispositif selon la revendication 10, **caractérisé par le fait que** l'extrémité de l'élément élastique opposée aux lanières est solidaire de la tige de piston.

12. Dispositif selon l'une quelconque des revendications précédentes, **caractérisé par le fait qu'**il comporte un élément de maintien (17) apte à passer par déformation d'une position de maintien du corps de la seringue (3) dans le fourreau (10) à une position dégagée permettant au corps de la seringue de coulisser librement par rapport au fourreau.

13. Dispositif selon la revendication 12, **caractérisé par le fait que** l'élément de maintien comporte des pattes élastiquement déformables (17) aptes à être écartées pour prendre la position dégagée.

14. Dispositif selon la revendication 13, **caractérisé par le fait que** l'élément élastique (15) comporte des ponts de matière (18) reliant les bras élastiquement déformables (16) auxdites pattes élastiquement déformables (17) de manière à ce que, Lorsque l'élément élastique subit une compression, lesdits ponts de matière tendent à écarter les pattes.

15. Dispositif selon la revendication 13, **caractérisé par le fait que** l'élément élastique (15) comporte une nervure annulaire (50) agencée pour venir en appui sur lesdites pattes (17) et les écarter lorsque l'élément élastique subit une compression.

16. Dispositif selon l'une quelconque des revendications précédentes, **caractérisé par le fait qu'**il comporte un élément de verrouillage (25) apte à être fixé sur le corps de la seringue (3) et agencé pour retenir celui-ci dans sa position de sécurité, après l'injection.

17. Dispositif selon la revendication 16, **caractérisé par le fait que** ledit élément de verrouillage comporte des dents (28) aptes à venir en butée contre un bord supérieur du fourreau de protection (10) lorsque le corps de la seringue est dans sa position de sécurité.

18. Dispositif selon l'une quelconque des revendications précédentes, dans lequel l'élément élastique (15) est apte à agir sur la seringue pendant le coulissement du corps de la seringue par rapport au fourreau depuis sa position d'injection jusqu'à sa position de sécurité pour l'amener dans sa position de sécurité après injection,
- l'une des extrémités (20) étant destinée à exercer une poussée sur le poussoir de la tige de piston et étant agencée de manière à pouvoir coulisser librement par rapport à cette tige de piston (7) avant l'injection, et/ou
- l'une des extrémités étant agencée de manière à pouvoir être fixée au fourreau de protection (10) dans une position réglable en hauteur.

## Claims

1. A safety device (2) for a prefilled syringe (1), the syringe comprising a body (3), a needle carrier (4) at one end of the body, a piston (6) movable inside the body, and a piston rod (7) projecting beyond the body at its end opposite from the needle carrier and suitable for pushing the piston inside the body towards the needle carrier, said device comprising a protective sheath (10) in which the syringe body can slide between an injection position in which the syringe needle extends outside the protective sheath, and a safe position in which the needle is withdrawn inside the protective sheath, **characterized in that** the device (2) comprises a resilient element (15) suitable for acting on the syringe to bring it into its safe position after injection, said resilient element having two axial ends and at least two elastically-deformable arms extending between said two axial ends, one of the ends (20) being arranged in such a manner as to be capable of sliding freely along the piston rod (7) prior to injection, and/or one of the ends being arranged so as to be capable of being fixed to the protective sheath (10) in a position of adjustable height.

2. A device according to claim 1, **characterized in that** each of the elastically-deformable arms (16) is defined by a strip of material made for example of PP, SBS, EBS, PA, POM, or PE, there preferably being two such arms disposed facing each other.

3. A device according to claim 2, **characterized in that** each of said arms (16) is generally V-shaped, with its concave sides facing inwards.

4. A device according to claim 3, **characterized in that** the branches of the V-shape of each of the arms (16) are of different lengths.

5. A device according to any one of claims 2 to 4, **characterized in that** the arms (16) present local reductions in width.

6. A device according to any one of previous claims, **characterized in that** the resilient element (15) is made integrally with the protective sheath (10).

7. A device according to claim 6, **characterized in that** one of the ends of the resilient element is connected to the protective sheath (10) and the other end is put into place on the piston rod (7), being free to slide relative thereto prior to injection.

8. A device according to claims 6 or 7, **characterized in that** the resilient element (15) includes an annular split ring (20) at one of its axial ends, which ring is suitable for engaging on the piston rod (7) and is capable of sliding relative thereto.

9. A device according to any one of claims 1 to 5, **characterized in that** the resilient element (15) is constituted by a separate element suitable for being fitted to the protective sheath (10).

10. A device according to claim 9, **characterized in that** the resilient element (15) includes fixing straps (49) each pierced by a plurality of orifices in a longitudinal direction of the strap, and the protective sheath includes portions in relief (55) each associated with one of the straps, each portion in relief being suitable for being fixed in one of the orifices of the associated strap.

11. A device according to claim 10, **characterized in that** the end of the resilient element remote from the strap is secured to the piston rod.

12. A device according to any one of previous claims, including a holding element (17) suitable for being deformed from a position for holding the syringe body (3) in the sheath (10) to a disengaged position enabling the syringe body to slide freely relative to the sheath.

13. A device according to claim 12, **characterized in that** the holding element comprises elastically-deformable tabs (17) suitable for being splayed apart in order to take up the disengaged position.

14. A device according to claim 13, wherein the resilient element (15) comprises bridges of material (18) connecting the elastically-deformable arms (16) to said elastically-deformable tabs (17) in such a manner that, when the resilient element is subjected to compression, said bridges of material tend to move the tabs apart.

15. A device according to claim 13, wherein the resilient element (15) comprises an annular rib (50) arranged to bear against said tabs (17) and move them apart when the resilient element is subjected to compression.

16. A device according to any one of previous claims, including a locking element (25) suitable for being fixed on the syringe body (3) and arranged to retain it in its safe position, after injection.

17. A device according to claim 16, **characterized in that** said locking element comprises teeth (28) suitable for coming into abutment against a top edge of the protective sheath (10) when the syringe body is in its safe position.

18. A device according to any one of previous claims, wherein the resilient element (15) is suitable for acting on the syringe during the sliding of the body of the syringe relative to the sheath from its injection position towards its safe position to bring it into its safe position after injection,
- one of the ends (20) being intended to exert a thrust on the pusher of the piston rod and being arranged in such a manner as to be capable of sliding freely along the piston rod (7) prior to injection, and/or
- one of the ends being arranged so as to be capable of being fixed to the protective sheath (10) in a position of adjustable height.

## Patentansprüche

1. Sicherheitsvorrichtung (2) für eine vorgefüllte Spritze (1), wobei letztere einen Körper (3), eine Nadelhalterung (4) an einem Ende dieses Körpers, einen bewegbaren Kolben (6) im Körper und einen Kolbenstift (7) umfasst, der an der Gegenseite der Nadelhalterung über den Körper hinausragt und geeignet ist, um den Kolben im Körper in Richtung der Nadelhalterung zu schieben, wobei besagte Vorrichtung eine Schutzhülle (10) aufweist, in welcher der Körper der Nadel gleiten kann zwischen einer Injektionsposition, bei der sich die Nadel der Spritze außerhalb der Schutzhülle erstreckt, und einer Sicherheitsposition, bei der die Nadel ins Innere der Schutzhülle zurückgezogen ist, **dadurch gekennzeichnet, dass** die Vorrichtung (2) ein elastisches Element (15) umfasst, das sich eignet, um auf die Spritze zu wirken, um sie nach der Injektion in ihre Sicherheitsposition zu führen, wobei dieses elastische Element zwei axiale Enden und wenigstens zwei elastisch verformbare Arme aufweist, die sich zwischen diesen beiden axialen Enden erstrecken, wobei eines der Enden (20) so angeordnet ist, dass es vor der Injektion in Bezug auf den Kolbenstift (7) ungehindert gleiten kann, und/oder eines der Enden so angeordnet ist, dass es an der Schutzhülle (10) in einer höhenverstellbaren Position angebracht werden kann.

2. Vorrichtung nach Anspruch 1, **dadurch gekennzeichnet, dass** die elastisch verformbaren Arme (16) jeweils durch ein Materialband, das beispielsweise aus PP, SBS, EBS, PA, POM, PE hergestellt ist, begrenzt sind und vorzugsweise zwei an der Zahl und einander gegenüber angeordnet sind.

3. Vorrichtung nach Anspruch 2, **dadurch gekennzeichnet, dass** besagte Arme (16) jeweils im Allgemeinen die Form eines V's aufweisen, dessen konkaver Teil nach innen gerichtet ist.

4. Vorrichtung nach Anspruch 3, **dadurch gekennzeichnet, dass** die Schenkel des V's jedes der Arme (16) unterschiedliche Längen aufweisen.

5. Vorrichtung nach einem der Ansprüche 2 bis 4, **dadurch gekennzeichnet, dass** die Arme (16) örtlich eine verringerte Breite aufweisen.

6. Vorrichtung nach einem der vorangehenden Ansprüche, **dadurch gekennzeichnet, dass** das elastische Element (15) in einem Stück mit der Schutzhülle (10) ausgeführt ist.

7. Vorrichtung nach Anspruch 6, **dadurch gekennzeichnet, dass** sich das eine der Enden des elastischen Elements an die Schutzhülle (10) anschließt und das andere der Enden auf dem Kolbenstift (7) angebracht ist, wobei es frei ist, in Bezug auf diesen zu gleiten, und zwar vor der Injektion.

8. Vorrichtung nach Anspruch 6 oder 7, **dadurch gekennzeichnet, dass** das elastische Element (15), an einem axialen Ende, einen gespaltenen Ring (20) aufweist, der in der Lage ist, sich an den Kolbenstift (7) anzufügen, und der in Bezug auf diesen gleiten kann.

9. Vorrichtung nach einem der Ansprüche 1 bis 5, **dadurch gekennzeichnet, dass** das elastische Element (15) durch ein angesetztes Element gebildet wird, das sich zur Befestigung auf der Schutzhülle (10) eignet.

10. Vorrichtung nach Anspruch 9, **dadurch gekennzeichnet, dass** das elastische Element (15) Befestigungsriemen (49) aufweist, die jeweils von einer Mehrzahl von Öffnungen durchbrochen sind, die einer Längsrichtung des Riemens folgen, und die Schutzhülle erhabene Stellen (55) aufweist, die jeweils zu einem Riemen gehören, wobei jede erhabene Stelle in einer der Öffnungen des zugehörigen Riemens befestigt werden kann.

11. Vorrichtung nach Anspruch 10, **dadurch gekennzeichnet, dass** das Ende des elastischen Elements, das den Riemen gegenüberliegt, solidarisch mit dem Kolbenstift ist.

12. Vorrichtung nach einem der vorangehenden Ansprüche, **dadurch gekennzeichnet, dass** diese ein Halteelement (17) aufweist, das sich eignet, um durch Verformung aus einer Position zum Halten des Körpers der Spritze (3) in der Hülle (10) zu einer gelösten Position überzugehen, die dem Körper der Spritze gestattet, in Bezug auf die Hülle ungehindert zu gleiten.

13. Vorrichtung nach Anspruch 12, **dadurch gekennzeichnet, dass** das Halteelement elastisch verformbare Halterungen (17) umfasst, die geeignet sind, um weggeschoben zu werden, um die gelöste Position einzunehmen.

14. Vorrichtung nach Anspruch 13, **dadurch gekennzeichnet, dass** das elastische Element (15) Materialbrücken (18) aufweist, welche die elastisch verformbaren Arme (16) mit den elastisch verformbaren Halterungen (17) verbinden, so dass, wenn das elastische Element einer Kompression ausgesetzt ist, besagte Materialbrücken dazu dienen, die Halterungen wegzuschieben.

15. Vorrichtung nach Anspruch 13, **dadurch gekennzeichnet, dass** das elastische Element (15) eine ringförmige Rippe (50) aufweist, die angeordnet ist, um sich auf besagte Halterungen (17) zu stützen und um sie wegzuschieben, wenn das elastische Element einer Kompression ausgesetzt ist.

16. Vorrichtung nach einem der vorangehenden Ansprüche, **dadurch gekennzeichnet, dass** diese ein Verriegelungselement (25) aufweist, das sich zur Befestigung auf dem Körper der Spritze (3) eignet und angeordnet ist, um diesen in seiner Sicherheitsposition zu halten, und zwar nach der Injektion.

17. Vorrichtung nach Anspruch 16, **dadurch gekennzeichnet, dass** besagtes Verriegelungselement Zähne (28) aufweist, die sich eignen, um gegen einen oberen Rand der Schutzhülle (10) anzuschlagen, wenn sich der Körper der Spritze in seiner Sicherheitsposition befindet.

18. Vorrichtung nach einem der Ansprüche 1 bis 17, bei der das elastische Element (15) in der Lage ist, während der Körper der Spritze in Bezug auf die Hülle von seiner Injektionsposition bis zu seiner Sicherheitsposition gleitet, auf die Spritze zu wirken, um sie nach der Injektion in ihre Sicherheitsposition zu führen,
- wobei eines der Enden (20) dazu bestimmt ist, einen Stoß auf das Teil zum Schieben des Kolbenstifts auszuüben, und so angeordnet ist, dass es vor der Injektion in Bezug auf diesen Kolbenstift (7) ungehindert gleiten kann, und/oder
- wobei eines der Enden so angeordnet ist, dass es an der Schutzhülle (10) in einer höhenverstellbaren Position angebracht werden kann.
